# EUROPEAN PATENT SPECIFICATION

(11) **EP 1 676 534 B1**
(45) Date of publication and mention of the grant of the patent: **26.03.2008**
(21) Application number: 05257962.0
(22) Date of filing: 22.12.2005
(51) Int. Cl.: A61B 17/00, A61K 47/02, A61L 24/00

(54) **Systems and methods for occluding a blood vessel**
System und Verfahren zum Gefässverschluss
Système et méthode pour l'obturation d'un vaisseau sanguin

(30) Priority: 29.12.2004 US 25229
(43) Date of publication of application: 05.07.2006
(73) Proprietor: Nitinol Development Corporation, Fremont, California 94539 (US)
(72) Inventor: Meretei, Attila, Fremont CA 94538 (US)
(74) Representative: Belcher, Simon James

(56) References cited:
- WO-A-20/04035022
- WO-A-20/04075989
- DE-A1- 4 327 219
- US-A- 5 951 600
- US-A1- 2004 224 864

## Description

The invention generally relates to systems and methods for occluding a blood vessel. More specifically, the invention relates to systems and methods for delivering and manipulating a variable viscosity fluid in the vascular system of a patient to provide a temporary occlusion of one or more blood vessels.

Endovascular occlusion, which involves closing off blood flow through one or more blood vessels in a patient, has most often been achieved using various non-surgical means. For instance, catheters can deliver various agents to block blood flow in the intended blood vessels. The various agents that are delivered to the intended blood vessels may be any of balloons, thrombosing (clogging) coils, sclerosing (hardening) drugs, and fast-acting embolization glues. While such endovascular occlusion techniques may be useful for occluding larger blood vessels, they often risk hemorrhage or damage to adjacent vessels or tissues. Further, such conventional techniques are not always conducive, or effective, for occluding smaller blood vessels, such as those found in capillary beds of a patient. Embolic compositions disclosed in WO 2004/075989 comprised fumed silica as a rheological modifier. The fumed silica imparts high viscosity to the compositions under static conditions but allows the compositions to flow readily under shear conditions.

Moreover, where capillary bleeding in the capillary bed occurs, typically no single capillary is responsible for the capillary bleeding. Rather any number of a plurality of capillaries within the capillary bed may be leaking. Moreover, the small size of capillaries renders it difficult to place traditional occluding devices therein without damaging the intended, or neighboring, capillaries or tissues. Thus, previously practiced techniques for individually occluding specifically targeted blood vessels are not always useful or conducive for occluding a plurality of the smaller and more delicate capillaries of a capillary bed.

In view of the above, a need exists for systems and methods that can more easily and safely occlude one or more blood vessels including the smaller more delicate capillary blood vessels of a capillary bed.

The systems and methods of the invention use a variable viscosity fluid delivered into the vascular system of a patient. The variable viscosity fluid is delivered into a targeted one or more blood vessels in a patient in order to occlude the targeted one or more blood vessels. The variable viscosity fluid is delivered into the vascular system of the patient in a liquefied state. The variable viscosity fluid may be delivered locally via a catheter or micro-catheter directly to the targeted one or more blood vessels to be occluded, or the variable viscosity fluid may be delivered indirectly to the targeted blood vessels by delivering the variable viscosity fluid to a feeder vessel that supplies the targeted one or more vessels to be occluded.

In any event, after reaching the targeted one or more blood vessels, the variable viscosity fluid is changed from its liquefied state to a solidified state in the targeted one or more blood vessels. The solidified variable viscosity fluid provides the intended occlusion to the targeted one or more blood vessels. Ideally, the variable viscosity fluid remains in its liquefied state in non-targeted blood vessels.

In the solidified state, the variable viscosity fluids occlude the targeted one or more blood vessels of the patient and restrict flow therethrough, whereas in the liquefied state the variable viscosity fluids freely flow through and permit other fluids, such as blood, to flow freely through the targeted blood vessels. The occlusions produced by the solidified state of the variable viscosity fluid may be temporary, of a duration as desired, within the targeted one or more blood vessels.

In some embodiments of the systems and methods of the invention, the variable viscosity fluid may be an electro-rheologic fluid, whereas in other embodiments the variable viscosity fluid may be a magneto-rheologic fluid. The variable viscosity fluid changes from the liquefied state to the solidified state according to the presence of an electric current in the case of an electro-rheologic fluid, or according to the presence of a magnetic field in the case of a magneto-rheologic fluid.

In those embodiments of the systems and methods of the invention using an electro-rheologic fluid, the systems and methods of the invention further provide an electric current generator. The electric current generator may be comprised of a power source connected to two or more electrodes placed on or about the targeted blood vessels to be occluded. The power source, when on, provides current to the electrodes so that a potential difference exists across the electrodes. The viscosity of the electro-rheologic fluids increases, thereby solidifying the electro-rheologic fluid, as the potential difference across the electrodes increases. The potential difference across the electrodes is thus generated by the power source, which may be internal or external of the patient, and is connected to the electrodes via wires.

In those embodiments of the systems and methods of the invention using a magneto-rheologic fluid, the systems and method of the invention further provide a magnetic field generator. The magnetic field generator is external of the patient and provides the magnetic field. The magnetic field generated by the magnetic field generator causes the magneto-rheologic fluid to solidify within the targeted blood vessels of the patient, thereby occluding the targeted blood vessels as intended. The magnetic field generator can be a permanent magnet, an electro-magnet, or a combination thereof. For convenience, the magnetic field generator may be a portable hand-held device easily manipulated by the medical professional over an area of the patient's body whereat the targeted blood vessels exist, or the magnetic field generator may temporarily secured to the patient's body whereat the targeted blood vessels exist. In either case, the targeted blood vessels are occluded as long as the magnetic field is present.

In those embodiments of the systems and methods of the invention using a magneto-rheologic fluid, a magnetic field generator system such as that described with respect to Figs. 7-10 in co-pending European Patent Application which claims priority from US Patent Application No. 11/025205, entitled "SYSTEMS AND METHODS FOR TREATING A THROMBUS IN A BLOOD VESSEL", may also be used to provide the magnetic field that changes the magneto-rheologic fluid between its liquefied and solidified states. The magnetic field generator may thus be portable or non-portable.

Where a catheter or micro-catheter is used to deliver magneto-rheologic fluids to the targeted one or more blood vessels, an otherwise conventional guide-wire may be provided with a magnetic tip. The magnetic tip of the guide-wire may be used to attract and withdraw magneto-rheologic fluids from the blood vessels after an occlusion made from the magneto-rheologic fluids is no longer needed. Alternatively, the magnetic tip of the guide-wire may be used to magnetically direct the magneto-rheologic fluids in the patient's body as desired.

The systems and methods of the invention thus provide a means for occluding blood vessels within the vasculature of a patient in an easy and convenient manner with minimal risk of damage to adjacent blood vessels or tissue. The systems and methods of the invention further provide temporary occlusions of variable durations within the intended blood vessels based on the presence or absence of one of an electric current or a magnetic field, depending on the type of variable viscosity fluid used. The systems and methods of the invention thus provide a relatively non-mechanical means of occluding one or more targeted blood vessels using the variable viscosity fluids, which fluids can be dispersed throughout the bloodstream, withdrawn, or re-captured when in their liquefied state.

The system of the present invention can be used in a method for occluding targeted one or more blood vessels in a patient, the method comprising:
delivering a variable viscosity fluid in a liquefied state to the targeted one or more blood vessels; and
changing the variable viscosity fluid from the liquefied state to a solidified state to occlude the targeted one or more blood vessels.

The above and other features of the invention, including various novel details of construction and combinations of parts, will now be more particularly described with reference to the accompanying drawings and claims. It will be understood that the various exemplary embodiments of the invention described herein are shown by way of illustration only and not as a limitation thereof. The principles and features of this invention may be employed in various alternative embodiments without departing from the scope of the invention.

Embodiments of the invention will now be described by way of example with reference to the accompanying drawings, in which:
Figure 1 illustrates generally the vascular anatomy of a capillary bed in a human;
Figure 2 illustrates a catheter or micro-catheter delivery of variable viscosity fluids to a human according to the invention;
Figure 3 illustrates schematically components used to change the state of an electro-rheologic fluid delivered to a human according to the invention;
Figure 4 illustrates schematically a hand-held magnetic field generator used to change the state of a magneto-rheologic fluid delivered to a human according to the invention;
Figure 5 illustrates schematically a portable magnetic tape for application to the body of a patient as the magnetic field generator according to the invention; and
Figure 6 illustrates a magnetic-tipped guide-wire usable with the catheter or micro-catheter of Figure 2 according to the invention.

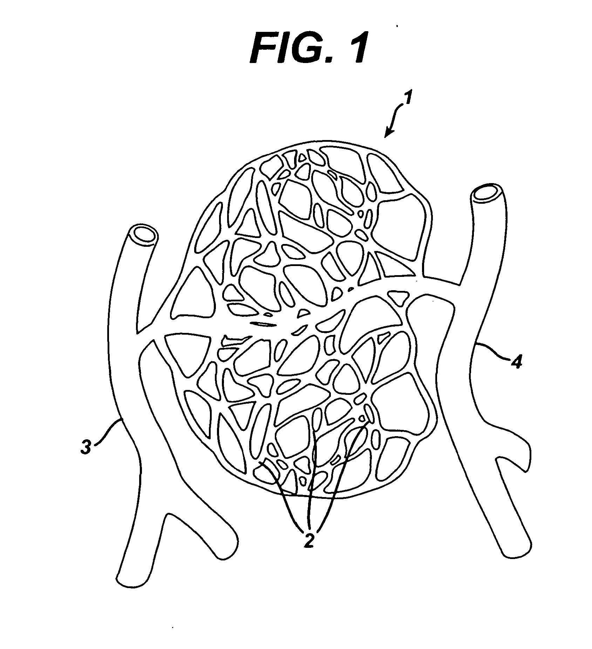

Fig. 1 illustrates generally the vascular anatomy of a capillary bed 1 in a human. The capillary bed 1 is comprised of capillaries 2 fed by arterioles 3 at one side of the bed and that feed venules 4 on the other side of the bed. The artisan will readily appreciate that the systems and methods described herein for occluding one or more blood vessels apply to human as well as non-human vasculatures, although the systems described herein are generally directed to occluding one or more targeted blood vessels in a human patient. Likewise, the artisan will readily appreciate that although the systems and methods of occluding one or more blood vessels as described herein are directed to occluding one or more capillaries in a capillary bed, blood vessels other than capillaries within the vasculature of a human or non-human patient may also be occluded using the systems and methods of the invention as described herein.

Referring still to Fig. 1, in the case of leaking capillaries 2 to be occluded in a capillary bed 1, a variable viscosity fluid is delivered to the capillaries 2 through an arteriole blood vessel 3 that generally supplies blood to all of the capillaries 2 of the capillary bed 1. Once the variable viscosity fluid reaches the targeted one or more capillaries 2 that are leaking in the capillary bed 1 the variable viscosity fluid is changed from a liquefied state to a solidified state. In this manner, the one or more leaking capillaries in the capillary bed may be occluded when the variable viscosity fluid is solidified with minimal risk of damage to surrounding capillaries or tissues in or near the capillary bed.

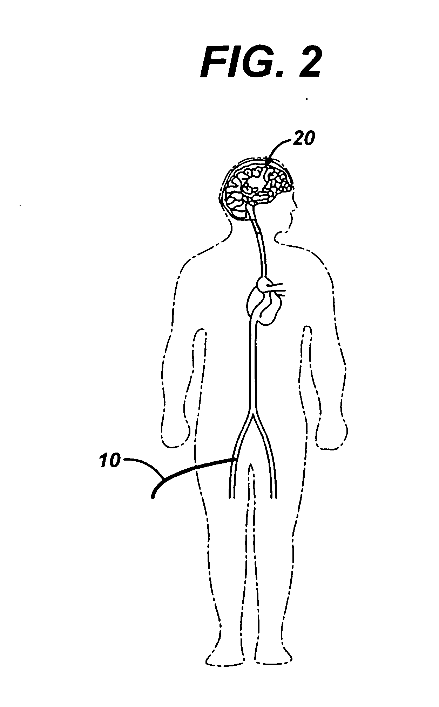

Fig. 2 illustrates generally the delivery of the variable viscosity fluids to the vasculature of a human using a catheter, or micro-catheter, 10. As shown in Fig. 2, the catheter, or micro-catheter, 10 delivers variable viscosity fluids locally to regions of the brain 20 for eventual occlusion of leaking capillaries, for example, therein. The artisan will appreciate, however, that because capillaries, or other blood vessels, are found in limbs and organs throughout the human, or other, anatomy, the catheter or micro-catheter 10 may be used to deliver the variable viscosity fluid to anatomical regions other than the brain in fairly conventional manner as well.

Preferably, the variable viscosity fluids are sufficiently radiopaque to be visualized using conventional fluoroscopy techniques, and are delivered directly to the targeted one or more blood vessels to be occluded using the catheter, or micro-catheter, 10. Alternatively, the variable viscosity fluids may be delivered indirectly to the targeted one or more blood vessels to be occluded by delivering the variable viscosity fluids to a feeder blood vessel, such as arteriole 3 that feeds the targeted capillaries 2 of Fig. 1. In either case, the variable viscosity fluids are eventually located at the targeted one or more blood vessels to be occluded.

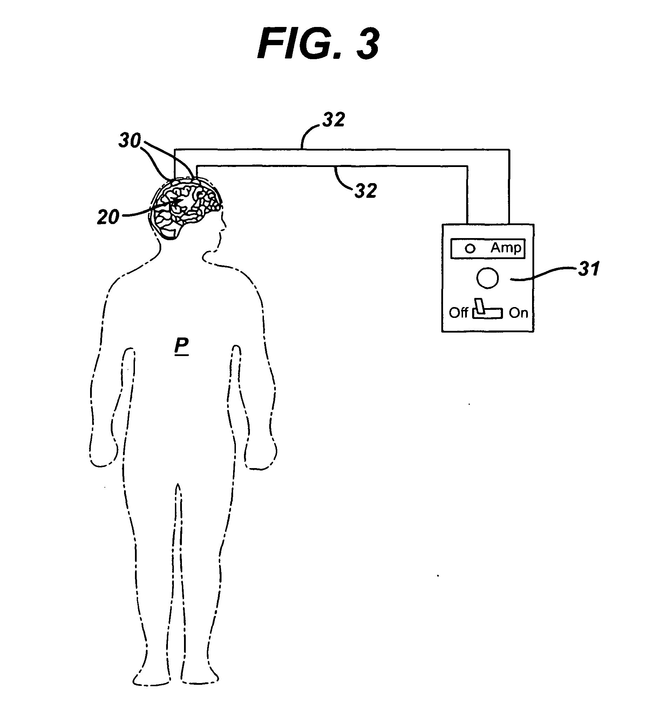

According to the systems and methods of the invention, the variable viscosity fluid may be an electro-rheologic fluid or a magneto-rheologic fluid. Fig. 3 illustrates schematically a case of an electro-rheologic fluid having been delivered to regions of the brain 20, wherein the delivery means is omitted for clarity from Fig. 3. More particularly, referring still to Fig. 3, electrodes 30 placed about the regions of the brain 20, whereat the targeted one or more blood vessels to be occluded are located, change the electro-rheologic fluid from the liquefied state to a solidified state when a sufficient potential difference between the electrodes 30 occurs. The amount of potential difference sufficient to solidify the electro-rheologic fluid can vary depending on the particular chemical composition of the electro-rheologic fluid and the anatomy within which it is used. The sufficiency of the potential difference can be verified by visualizing the distribution and movement of the electro-rheologic fluid fluoroscopically within the targeted one or more blood vessels, for example, by intravascular viscosity measurements. When solidified, the electro-rheologic fluid creates the intended occlusion in the targeted one or more blood vessels. The artisan will appreciate that the electrodes can be placed at other regions of the anatomy to target other blood vessels to be occluded. The occlusions are temporary, and of a duration determined by the presence of the potential difference between the electrodes 30, as desired.

Referring still to Fig. 3, an electric current generator, or power source 31, provides current that generates the potential difference between the electrodes. The duration of the occlusion depends on the presence of the potential difference between the electrodes. The electrodes are ideally placed on or near the targeted one or more blood vessels so as to have the most direct effect on the electro-rheologic fluid contained within the targeted one or more vessels.

An electric current generator, or power source, 31 is provided internally or externally of the patient to generate the electric current that creates the potential difference between the at least two electrodes that induces the electro-rheologic fluid to change states. In the absence of a sufficient potential difference between the at least two electrodes, the electro-rheologic fluid resumes its liquefied state. Terminating the supply of current from the electric current generator, or power source, 31 thus removes the occlusion in the targeted blood vessels and the liquefied electro-rheologic fluid is washed away by the blood flow within the blood vessels of the patient. The electric current generator, or power source, 31 may be wired to the at least two electrodes using wires 32, as shown in Fig. 3.

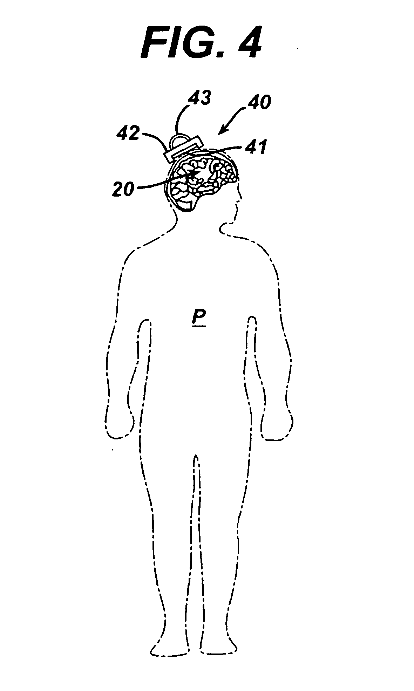

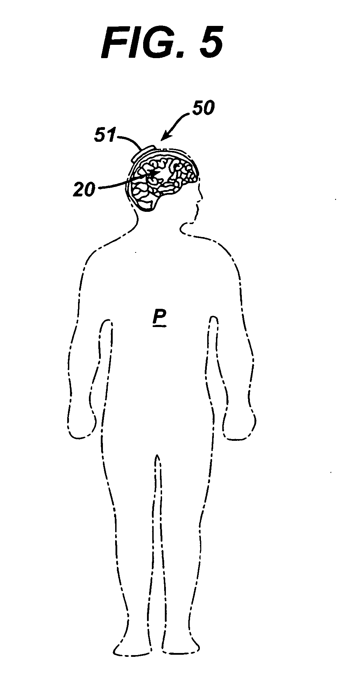

Figs. 4 and 5 illustrate schematically the case of a magneto-rheologic fluid having been delivered to regions of the brain 20, wherein the delivery means is omitted for clarity. As shown in Figs. 4 and 5, the generation of a magnetic field by a magnetic field generator changes the magneto-rheologic fluid within the targeted one or more blood vessels to its solidified state. In this manner, the intended occlusion of the targeted blood vessels occurs.

More specifically, referring to Fig. 4, a magnetic field generator comprises a portable hand-held member 40 having at least one magnet 41, a housing 42, and a handle 43. The at least one magnet 41 is located within the housing 42, for example, as shown in Fig. 4, although the artisan will appreciate that the housing 42 could instead be omitted, in which case the handle 43 would extend directly from the at least one magnet 41. In practice, the hand-held member 40 is positioned to locate the at least one magnet 41 over the body part of the patient P whereat the targeted one or more blood vessels to be occluded are located. In Fig. 4, the hand-held member 40 is positioned to locate the at least one magnet 41 over that area of the brain 20 whereat the capillary bed blood vessels are to be occluded, the capillary bed blood vessels already having received the magneto-rheologic fluid in its liquefied state. By the presence of the magnetic field generated by the at least one magnet 41 of the hand-held device 40, the magneto-rheologic fluid solidifies to provide the intended occlusion. The hand-held member 40 may be moved, in the discretion of the medical professional, in order to position the at least one magnet 41 in a more appropriate orientation to occlude the targeted one or more blood vessels, or to remove the hand-held device 40 from the patient to terminate the occlusion and return the magneto-rheologic fluid to its liquefied state.

Referring to Fig. 5, the magnetic field generator is a magnetic tape 50 comprised of at least one magnet 51. Rather than the hand-held device 40 of Fig. 4 that requires the medical professional to hold the at least one magnet 41 in place over the patient P, the magnetic tape 50 of Fig. 5 may be attached to the body part of the patient P whereat the targeted blood vessels to be occluded are located. In this manner, occlusions of longer duration may be more conveniently achieved using the magnetic tape 50 of Fig. 5 than by using the hand-held device 40 of Fig. 4. In all other respects, the magnetic tape 50 and at least one magnet 51 of Fig. 5 acts in much the same way as the hand-held device 40 and at least one magnet 41 of Fig. 4, whereby removal of the magnetic tape 50 and the at least one magnet 51 also terminates the occlusions and returns the magneto-rheologic fluid to its liquefied state. Thus, the magnetic tape 50 of Fig. 5 renders occlusions of longer duration more easily and conveniently achieved and enables the patient P to leave a medical facility or to leave the immediate observation of the medical professional if permitted, which is not as readily available using the hand-held device 40 of Fig. 4.

Of course, referring to the magnetic field generators of Figs. 4 and 5, the artisan should readily appreciate that although the at least one magnet 41 shown in Fig. 4 is a single magnet, the magnetic field may instead be generated by a plurality of magnets. Moreover, where used, the plurality of magnets may be arranged in a variety of configurations. The at least one magnet may be a permanent magnet, an electro-magnet, or some combination thereof. In any event, a position of the at least one magnet, or a position of the patient relative to the at least one magnet, may be altered in order to alter the strength, geometry or gradient of the magnetic field generated thereby. Where a plurality of magnets are provided as the at least one magnet, changes in the strength and geometry of the magnetic field may be achieved by individually tuning the strength of the individual electro-magnets, or by sequencing their operation so that some magnets are on, while other magnets are off at any given time.

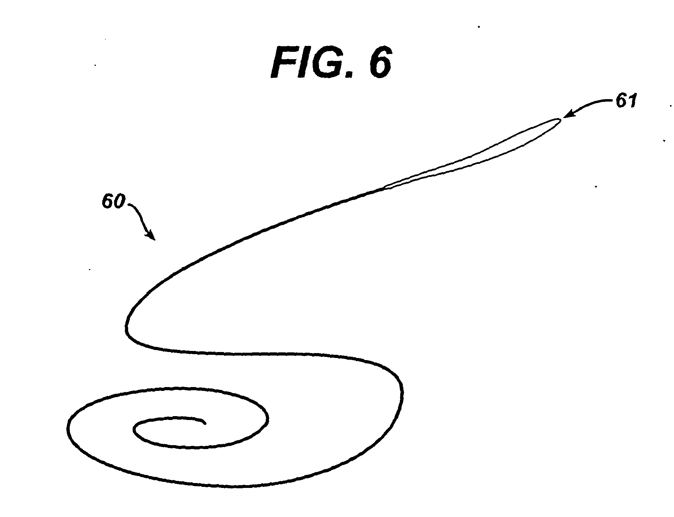

The liquefied fluid may either remain in the blood stream of the patient, or, where as shown in Fig. 6, an otherwise conventional guide-wire 60 having a magnetic tip 61 is used with the catheter or micro-catheter 10, as described above, the liquefied magneto-rheologic fluid may be magnetically re-captured and withdrawn from the patient.

In the various embodiments of the systems and methods of the invention, the blood vessel occlusions provided by the variable viscosity fluid are temporary, whereby the electric current or magnetic field induces the variable viscosity fluid disposed within the targeted blood vessels to solidify only as long as the electric current or magnetic field is present. The duration of the occlusion is thus variable according to the presence or absence of the electric current or magnetic field. When the occlusions are no longer needed and the solidified fluid is returned to its liquefied state by terminating the electric current or magnetic to remove the occlusion, the liquefied variable viscosity fluid is then washed away through the re-opened blood vessel or vessels in the normal course of blood flow therethrough. Alternatively, where magneto-rheologic fluids are used, the liquefied magneto-rheologic fluids may be magnetically re-captured and withdrawn from the blood vessels as discussed above.

Ideally, any variable viscosity fluids in blood vessels not intended to be occluded will remain liquefied by not directing an electric current or magnetic field to those non-intended blood vessels. As a result, those non-intended blood vessels should remain open to permit the passage of the variable viscosity fluids, as well as other fluids such as blood, therethrough. If a non-intended blood vessel is inadvertently occluded by the generation or presence of an electric current or a magnetic field, then the inadvertent occlusion is readily removed by turning off, repositioning or otherwise removing the electric current or magnetic field from the proximity of the targeted blood vessels. In this manner, and in the absence of the potential difference or magnetic field, the variable viscosity fluid returns to its liquefied state as discussed above.

Because the variable viscosity fluids are preferably sufficiently radiopaque to be visualized using conventional fluoroscopy techniques, the activity of the variable viscosity fluids may be readily viewed to determine the status of the targeted blood vessels. In this manner, a medical professional may determine whether or not the induced occlusions have sufficiently stopped leakages in the targeted one or more blood vessels, or whether an occlusion of longer duration is needed, or whether an occlusion of longer length is needed within a blood vessel.

The various exemplary embodiments of the invention as described hereinabove do not limit different embodiments of the systems and methods of the invention. The material described herein is not limited to the materials, designs or shapes referenced herein for illustrative purposes only, and may comprise various other materials, designs or shapes suitable for the systems and methods described herein, as should be appreciated by the artisan.

## Claims

1. A system for occluding a targeted one or more blood vessels in a patient, the system comprising:
a variable viscosity fluid disposed within the targeted one or more blood vessels; and
means for changing the variable viscosity fluid to a solidified state from a liquefied state, the solidified state of the variable viscosity fluid occluding the targeted one or more blood vessels.

2. The system of claim 1, wherein the variable viscosity fluid is one of an electro-rheologic fluid or a magneto-rheologic fluid.

3. The system of claim 2, further comprising:
means for delivering the variable viscosity fluid to the targeted one or more blood vessels of the patient.

4. The system of claim 3, wherein the means for delivering delivers the variable viscosity fluid comprises one of a catheter or a micro-catheter (10) that delivers the variable viscosity fluid to the targeted one or more blood vessels or to a vessel that feeds the targeted one or more blood vessels.

5. The system of claim 3, further comprising:
at least two electrodes (30) attached to the patient on or near the targeted one or more blood vessels; and
an electric current generator (31) connected to the at least two electrodes, the electric current generator providing current that creates a potential difference between the at least two electrodes to solidify the electro-rheologic fluid.

6. The system of claim 5, wherein the electric current generator (31) further comprises wires (32) connecting the electric current generator to the at least two electrodes (30).

7. The system of claim 5, wherein the electric current generator (31) is external of the patient.

8. The system of claim 5, wherein the electric current generator (31) is implanted in the patient.

9. The system of claim 3, further comprising:
a magnetic field generator having at least one magnet that generates to solidify the magneto-rheologic fluid when the magnetic field generator is placed over the body part whereat the targeted one or more blood vessels are located.

10. The system of claim 9, wherein the magnetic field generator is external of the patient.

11. The system of claim 9, wherein the at least one magnet is a plurality of magnets.

12. The system of claim 9, wherein the at least one magnet is a permanent magnet, an electro-magnet, or a combination thereof.

13. The system of claim 10, wherein the magnetic field generator is a flexible magnetic tape securable to the patient at the body part whereat the targeted one or more blood vessels are located.

14. The system of claim 10, further comprising:
at least one magnet (41), and
a handle (43) connected to the at least one magnet, the at least one magnet being oriented on the patient relative to the targeted one or more blood vessels to solidify the magneto-rheologic fluid.

15. The system of claim 14, further comprising:
a housing (42) within which the at least one magnet is housed, the handle being connected to the housing.

16. The system of claim 10, wherein the means for delivering the variable viscosity fluid further comprises a magnetic tip guide-wire (60) in the catheter or micro-catheter (10), the magnetic tip guide-wire assisting direction or withdrawal of the magneto-rheologic fluid in the patient.

17. The system of claim 4, wherein the targeted one or more blood vessels are capillaries in a capillary bed of the patient.

18. The system of claim 1, wherein the variable viscosity fluid is sufficiently radiopaque to be fluoroscopically visualized.

## Patentansprüche

1. System zum Verschließen eines oder mehrerer Blutgefäße, die als Ziel gesetzt sind, bei einem Patienten, wobei das System aufweist:
ein Fluid mit variabler Viskosität, das innerhalb der einen oder der mehreren Blutgefäße, die als Ziel gesetzt sind, angeordnet ist; und
eine Einrichtung zum Ändern des Fluides mit variabler Viskosität aus einem verflüssigten Zustand in einen verfestigten Zustand, wobei der verfestigte Zustand des Fluides mit variabler Viskosität das eine oder die mehreren Blutgefäße, die als Ziel gesetzt sind, verschließt.

2. System nach Anspruch 1, bei dem das Fluid mit variabler Viskosität entweder ein e-lektrorheologisches Fluid oder ein magnetorheologisches Fluid ist.

3. System nach Anspruch 2, das weiter aufweist:
eine Einrichtung zum Zuführen des Fluides mit variabler Viskosität zu dem einen oder den mehreren Blutgefäßen, die als Ziel gesetzt sind.

4. System nach Anspruch 3, bei dem die Einrichtung zum Zuführen, die das Fluid mit variabler Viskosität zuführt, entweder einen Katheter oder einen Mikrokather (10) aufweist, der Fluid mit variabler Viskosität an das eine oder die mehreren Blutgefäße, die als Ziel gesetzt sind, oder an ein Gefäß, das das eine oder die mehreren Blutgefäße, die als Ziel gesetzt sind, speist, liefert.

5. System nach Anspruch 3, das weiter aufweist:
wenigstens zwei Elektroden (30), die an dem Patienten auf oder nahe dem einen oder den mehreren Blutgefäßen, die als Ziel gesetzt sind, befestigt sind; und
einen Generator (31) für elektrischen Strom, der mit den wenigstens zwei Elektroden verbunden ist, wobei der Generator für elektrischen Strom Strom liefert, der eine Potentialdifferenz zwischen den wenigstens zwei Elektroden erzeugt, um das elektrorheologische Fluid zu verfestigen.

6. System nach Anspruch 5, bei dem der Generator (31) für elektrischen Strom weiter Drähte (32) aufweist, die den Generator für elektrischen Strom mit den wenigstens zwei Elektroden (30) verbinden.

7. System nach Anspruch 5, bei dem der Generator (31) für elektrischen Strom sich außerhalb des Patienten befindet.

8. System nach Anspruch 5, bei dem der Generator (31) für elektrischen Strom in den Patienten implantiert ist.

9. System nach Anspruch 3, das weiter aufweist:
einen Generator für ein Magnetfeld, der wenigstens einen Magneten hat, welcher generiert, um das magnetorheologische Fluid zu verfestigen, wenn der Generator für ein magnetisches Feld über den Körperbereich gebracht wird, in dem sich das eine oder die mehreren Blutgefäße, die als Ziel gesetzt sind, befinden.

10. System nach Anspruch 9, bei dem sich der Generator für ein magnetisches Feld außerhalb des Patienten befindet.

11. System nach Anspruch 9, bei dem der wenigstens eine Magnet eine Vielzahl von Magneten ist.

12. System nach Anspruch 9, bei dem der wenigstens eine Magnet ein Dauermagnet, ein Elektromagnet oder eine Kombination aus diesen ist.

13. System nach Anspruch 10, bei dem der Generator für ein Magnetfeld ein flexibles Magnetband ist, das an dem Patienten an dem Körperbereich zu sichern ist, wo sich die einen oder mehreren Blutgefäße, die als Ziel gesetzt sind, befinden.

14. System nach Anspruch 10, das weiter aufweist:
wenigstens einen Magneten (41); und
einen Griff (43), der mit dem wenigstens einen Magneten verbunden ist, wobei der wenigstens eine Magnet auf dem Patienten relativ zu dem einen oder den mehreren Blutgefäßen, die als Ziel gesetzt sind, so ausgerichtet wird, daß sich das magnetorheologische Fluid verfestigt.

15. System nach Anspruch 14, das weiter aufweist:
ein Gehäuse (42), in dem der wenigstens eine Magnet untergebracht ist, wobei der Griff mit dem Gehäuse verbunden ist.

16. System nach Anspruch 10, bei dem die Einrichtung zum Zuführen des Fluides mit variabler Viskosität weiter einen magnetischen Spitzenfiikwuxgsdraht (60) in dem Katheter oder dem Mikrokatheter (10) aufweist, wobei der magnetische Spitzenfiihrungsdraht beim Führen oder Zurückziehen des magnetorheologischen Fluides in dem Patienten unterstützt.

17. System nach Anspruch 4, bei dem die einen oder mehreren Blutgefäße, die als Ziel gesetzt sind, Kapillaren in einem kapillaren Bett des Patienten sind.

18. System nach Anspruch 1, bei dem das Fluid mit variabler Viskosität ausreichend strahlungsuudurchlassig ist, so daß es fluoroskopisch sichtbar ist.

## Revendications

1. Système destiné à obturer un ou plusieurs vaisseau(x) sanguin(s) ciblé(s) chez un patient, le système comprenant :
◆ un fluide à viscosité variable disposé à l'intérieur du ou des vaisseau(x) sanguin(s) ciblé(s) ; et
◆ des moyens destinés à modifier le fluide à viscosité variable vers un état solidifié depuis un état liquéfié, l'état solidifié du fluide à viscosité variable obturant le ou les vaisseau(x) sanguin(s) ciblé(s).

2. Système selon la revendication 1, dans lequel le fluide à viscosité variable est un parmi un fluide électrorhéologique ou un fluide magnétorhéologique.

3. Système selon la revendication 2, comprenant en outre :
◆ des moyens destinés à distribuer le fluide àviscosité variable vers le ou les vaisseau(x) sanguin(s) ciblé(s) du patient.

4. Système selon la revendication 3, dans lequel les moyens de distribution du fluide à viscosité variable comprennent un parmi un cathéter ou un micro-cathéter (10) qui distribue le fluide à viscosité variable au ou aux vaisseau(x) sanguin (s) ciblé(s) ou à un récipient qui alimente le ou les vaisseau(x) ciblé(s).

5. Système selon la revendication 3, comprenant en outre :
◆ au moins deux électrodes (30) fixées au patient sur le ou les ou à proximité du ou des vaisseau(x) sanguin(s) ciblé (s) ; et
◆ un générateur de courant électrique (31) connecté à les au moins deux électrodes, le générateur de courant électrique fournissant un courant qui crée une différence de potentiel entre les au moins deux électrodes pour solidifier le fluide électrorhéologique.

6. Système selon la revendication 5, dans lequel le générateur de courant électrique (31) comprend en outre des fils (32) connectant le générateur de courant électrique à les au moins deux électrodes (30).

7. Système selon la revendication 5, dans lequel le générateur de courant électrique (31) se situe à l'extérieur du patient.

8. Système selon la revendication 5, dans lequel le générateur de courant électrique (31) est implanté dans le patient.

9. Système selon la revendication 3, comprenant en outre :
◆ un générateur de champ magnétique présentant au moins un aimant qui génère pour solidifier le fluide magnétorhéologique lorsque le générateur de champ magnétique est placé au-dessus de la partie de corps dans laquelle le ou les vaisseau(x) sanguin(s) ciblé(s) sont situé(s).

10. Système selon la revendication 9, dans lequel le générateur de champ magnétique se situe à l'extérieur du patient.

11. Système selon la revendication 9, dans lequel le au moins un aimant est une pluralité d'aimants.

12. Système selon la revendication 9, dans lequel le au moins un aimant est un aimant permanent, un électro-aimant, ou leur combinaison.

13. Système selon la revendication 10, dans lequel le générateur de champ magnétique est une bande magnétique flexible pouvant être fixée au patient au niveau de la partie du corps au niveau de laquelle le ou les vaisseau(x) sanguin(s) ciblé(s) est(sont) situé(s).

14. Système selon la revendication 10, comprenant en outre :
◆ au moins un aimant (41), et
◆ une poignée (43) connectée à le au moins un aimant, le au moins un aimant étant orienté sur le patient par rapport au ou aux vaisseau(x) sanguin(s) ciblé(s) pour solidifier le fluide magnétorhéologique.

15. Système selon la revendication 14, comprenant en outre :
◆ un boîtier (42) à l'intérieur duquel le au moins un aimant est logé, la poignée étant connectée au boîtier.

16. Système selon la revendication 10, dans lequel les moyens destinés à distribuer le fluide à viscosité variable comprennent un fil-guide à pointe magnétique (60) dans le cathéter ou micro-cathéter (10), le guide-fil à pointe magnétique aidant à diriger ou à retirer le fluide magnétorhéologique dans le patient.

17. Système selon la revendication 4, dans lequel le ou les vaisseau(x) sanguin(s) ciblé(s) sont des capillaires dans un lit capillaire du patient.

18. Système selon la revendication 1, dans lequel le fluide à viscosité variable est suffisamment radio-opaque pour être visualisé de manière fluoroscopique.
